# DEMANDE DE BREVET EUROPEEN

(11) **EP 0 606 031 A1**
(43) Date de publication de la demande: **13.07.1994**
(21) Numéro de dépôt: 93403138.6
(22) Date de dépôt: 22.12.1993
(51) Int. Cl.: A61K 31/165, A61K 31/615, A61K 9/00

(54) **Poudre pour solution buvable à base d'acétylsalicylate de lysine et de métoclopramide**

(30) Priorité: 04.01.1993 FR 9300008
(71) Demandeur: SYNTHELABO, F-92350 Le Plessis Robinson (FR)
(72) Inventeur: Depauw, Thierry, F-37230 Fondettes (FR); Senequier, Raymond, F-37540 Saint Cyr sur Loire (FR)
(74) Mandataire: Thouret-Lemaitre, Elisabeth

(57) **Abrégé**

Poudre pour solution buvable caractérisée par le fait qu'elle contient un mélange d'acétylsalicylate de D,L-lysine et de chlorhydrate de métoclopramide, en association avec des excipients appropriés.

Application thérapeutique

## Description

La présente invention a pour objet une poudre pour solution buvable à base d'acétylsalicylate de lysine et de chlorhydrate de métoclopramide destinée à être présentée en sachet.

La combinaison de métoclopramide et d'un agent analgésique, par exemple, le paracétamol, sous forme de comprimé, a déjà été décrite dans le brevet européen EP 0011490.

La combinaison de métoclopramide et d'acide acétylsalicylique sous la forme de comprimé effervescent est également connue.

La demanderesse a mis au point une poudre pour sachet contenant un mélange d'acétylsalicylate de D,L-lysine et de chlorhydrate de métoclopramide.

Le chlorhydrate de métoclopramide est de préférence le monochlorhydrate monohydraté.

L'acétylsalicylate de D,L-lysine est un agent analgésique bien connu, soluble dans l'eau, stable, qui améliore considérablement la tolérance digestive locale de l'acide acétylsalicylique.

Le monochlorhydrate de métoclopramide est également un agent thérapeutique bien connu.

L'administration par voie orale de la poudre pour solution buvable contenant l'association de l'invention permet de traiter efficacement la migraine.

Les compositions de l'invention peuvent contenir l'équivalent de 450 à 1350 mg d'acide acétylsalicylique et de 5 à 15 mg de monochlorhydrate de métoclopramide.
La composition comprend avantageusement 1620 mg d'acétylsalicylate de lysine et 10,54 mg de monochlorhydrate monohydraté de métoclopramide ; soit l'équivalent de 900 mg d'acide acétylsalicylique et de 10 mg de monochlorhydrate de métoclopramide.

La poudre pour solution buvable peut contenir d'autres composants utilisés habituellement lors de la formulation et de la fabrication de ces poudres, tels que des lubrifiants, par exemple la glycine, des aromatisants et des édulcorants.

Il est indispensable qu'un mélange homogène des constituants soit obtenu par une compatibilité granulométrique entre les composants.

Par conséquent, il s'avère que la granulométrie des composants doit être la suivante :
- acétylsalicylate de lysine : diamètre moyen 80 microns
- monochlorhydrate monohydraté de métoclopramide : diamètre moyen inférieur à 100 microns.

Le procédé d'obtention de la poudre pour solution buvable selon l'invention doit être réalisé de manière à obtenir une très bonne homogénéité du mélange des composants.

Lorsque le mélange de composants est obtenu, il est réparti dans des sachets.
Un exemple de poudre pour solution buvable est le suivant :
- acétylsalicylate de DL-lysine 1620,00 mg
- monochlorhydrate monohydraté de métoclopramide 10,54 mg
- glycine 179,46 mg
- arôme citron 30,00 mg
- aspartam 20,00 mg

## Revendications

1. Poudre pour solution buvable caractérisée par le fait qu'elle contient un mélange d'acétylsalicylate de D,L-lysine et de chlorhydrate de métoclopramide, en association avec des excipients appropriés.

2. Poudre pour solution buvable selon la revendication 1, caractérisée par le fait que le chlorhydrate de métoclopramide est le monochlorhydrate monohydraté de métoclopramide.

3. Poudre pour solution buvable selon la revendication 1 ou la revendication 2, caractérisée par le fait qu'elle contient les constituants acétylsalicylate de lysine et monochlorhydrate monohydraté de métoclopramide en quantités telles qu'il est délivré, par sachet-dose, de 450 à 1350 mg d'acide acétylsalicylique et de 5 à 15 mg de monochlorhydrate de métoclopramide.

4. Poudre pour solution buvable selon l'une quelconque des revendications 1 à 3, caractérisée par le fait qu'elle contient les constituants acétylsalicylate de lysine et monochlorhydrate monohydraté de métoclopramide en quantités telles qu'il est délivré, par sachet-dose, 900 mg d'acide acétylsalicylique et 10 mg de monochlorhydrate de métoclopramide.

5. Poudre pour solution buvable selon l'une quelconque des revendications 1 à 4, caractérisée par le fait qu'elle contient comme excipients, un ou plusieurs lubrifiants, un ou plusieurs aromatisants et un ou plusieurs édulcorants.

6. Poudre pour solution buvable, selon l'une quelconque des revendications 1 à 5, caractérisée par le fait que la granulométrie des constituants est la suivante :
- acétylsalicylate de lysine : diamètre moyen 80 microns
- monochlorhydrate monohydraté de métoclopramide : diamètre moyen inférieur à 100 microns.

7. Procédé de préparation de la poudre pour solution buvable, définie dans l'une quelconque des revendications 1 à 6, procédé caractérisé en ce que l'on mélange de manière homogène les divers constituants.
